(19)

**Europäisches Patentamt**

**European Patent Office**

**Office européen des brevets**

(11) **EP 2 937 040 B1**

(12) **EUROPEAN PATENT SPECIFICATION**

(45) Date of publication and mention
of the grant of the patent:
**02.08.2023 Bulletin 2023/31**

(21) Application number: **14165582.9**

(22) Date of filing: **23.04.2014**

(51) International Patent Classification (IPC):
**A61B 5/12** *(2006.01)* **A61B 5/00** *(2006.01)*

(52) Cooperative Patent Classification (CPC):
**A61B 5/126;** A61B 2560/0266; A61B 2562/0204

(54) **Audiologic test apparatus and related method**

Audiologische Testvorrichtung und entsprechendes Verfahren

Appareil de test audiophonatoire et procédé associé

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**28.10.2015 Bulletin 2015/44**

(73) Proprietor: **Natus Medical Incorporated
Pleasanton, CA 94566 (US)**

(72) Inventor: **Reinholdt-Nielsen, Christian
DK-3600 Frederikssund (DK)**

(74) Representative: **Zacco Denmark A/S
Arne Jacobsens Allé 15
2300 Copenhagen S (DK)**

(56) References cited:
WO-A1-98/06324     JP-A- 2001 149 347
US-A- 5 577 511     US-A- 5 825 894
US-B1- 7 050 592

**Description**

The present disclosure relates to a method, a system and an apparatus for initiating and/or performing an audiologic test, such as tympanometry and/or an otoacoustic emission test, in an ear canal of a person. In particular, an audiologic test apparatus, system and method for performing an audiologic test is disclosed. The audiologic test may comprise a tympanometric and/or an otoacoustic emission test.

BACKGROUND

Audiologic tests, such as tympanometry and/or an otoacoustic emission test, examines the condition of the middle ear, mobility of the tympanic membrane, and/or the conduction bones by creating variations of pressure in the ear canal. In order to modify the pressure, a probe is inserted into the ear canal creating an air tight seal of the ear canal.

Audiologic tests are conventionally performed by transmitting a continuous tone with a primary frequency component at a primary frequency of 226 Hz towards the tympanic membrane and measuring via a microphone the signal reflected by the tympanic membrane. The choice of 226 Hz is a de facto standard in performing a tympanometry in adults. In small children a tympanometry is occasionally performed with a tone with a primary frequency component at a primary frequency about 1,000 Hz.

Conventionally apparatuses configured to perform audiologic tests incorporate measures to automatically detect if the probe is inserted into the ear. This is conventionally achieved by transmitting, through the test probe, the test tone, e.g. 226 Hz, whenever the apparatus is switched on. The microphone detects a reflection of the test tone, and the reflection is an indicator that the test probe is inserted into an ear canal. However, the reflection may indicate that the test probe is inserted into an ear canal, even if an air tight seal is not created. Hence, the apparatus may activate a pump to increase/decrease pressure even if the test probe is not correctly positioned in or at the ear canal.

US 5,577,511 discloses an instrument for measuring the degree of occlusion of an occluding object, such as an earmold, in the ear canal of a subject. The instrument includes a first microphone for transducing sound waves exterior to the ear canal into electrical signals and a first microphone for transducing sound waves within the ear canal into electrical signals.

US 7,050,592 discloses a hearing test device and a method which involves the placement of a testing probe in the ear canal of a test subject. The device analyzes responses to stimuli applied to the ear canal to determine whether the testing probe has been properly placed in the ear canal. If the device determines that the testing probe has been properly placed in the ear canal, the device automatically starts a hearing test without requiring any operator input.

SUMMARY

Despite the known solutions there is still a need for a system, apparatus and/or a method which decrease the risk of falsely initiating an audiologic test.

Accordingly, an audiologic test apparatus for performing an audiologic test in an ear canal, is provided. The audiologic test apparatus comprises a housing, a processing unit, a tone generator connected to the processing unit, and a probe interface for connecting the audiologic test apparatus to a test probe. The apparatus is configured to: generate by the tone generator a first electrical signal representative of a first signal with a first primary frequency component at a first primary frequency that is within a range from 30 Hz to 80 Hz; receive or obtain a first response signal; determine if a first insertion criterion is satisfied, wherein the first insertion criterion is based on the first signal and/or the first response signal; and initiate the audiologic test if at least the first insertion criterion is satisfied. The audiologic test comprises generating a second electrical signal representative of a second signal with a second primary frequency component at a second primary frequency. The first primary frequency is lower than the second primary frequency.

Also disclosed is an audiologic test system for performing an audiologic test, the audiologic test system comprising: an audiologic test apparatus, such as the disclosed audiologic test apparatus, the audiologic test apparatus comprising a processing unit; a test probe with a first part for insertion into an ear canal, the test probe being connected to the audiologic test apparatus; a first speaker; and a first microphone. The audiologic test system is configured to: generate a first signal with a first primary frequency component at a first primary frequency that is within a range from 30 Hz to 80 Hz; receive a first response signal; determine if a first insertion criterion is satisfied, wherein the first insertion criterion is based on the first signal and/or the first response signal; and initiate the audiologic test if at least the first insertion criterion is satisfied. The audiologic test comprises generating a second signal with a second primary frequency component at a second primary frequency. The first primary frequency is lower than the second primary frequency.

Also disclosed is a method for performing and/or initiating an audiologic test. The method may comprise performing one or more insertion tests including a first insertion test and/or a second insertion test, e.g. before and/or during the audiologic test. The method may comprise, e.g. as a part of the first insertion test, generating a first signal with a first primary frequency component at a first frequency that is within a range from 30 Hz to 80 Hz; detecting a first response signal; determining if a first insertion criterion is satisfied, wherein the first insertion criterion is based on the first signal and/or the first response signal. The method may comprise initiating the audiologic test if at least the first insertion criterion is satisfied and/or if one or more insertion tests are passed. The audiologic test comprises generating a second signal with a second primary frequency component at a second primary frequency. The first primary frequency is lower than the second primary frequency.

The disclosed apparatus, system and method provide improved reliability of automation in audiologic test apparatuses and/or systems. The risk of activating unnecessary steps and/or unnecessary activation of mechanical parts is reduced.

Furthermore, the disclosed apparatus, system and method advantageously provide for faster test procedures, e.g. unsuccessful attempts may be avoided.

An even further advantage of the disclosed apparatus, system and method is that wear of mechanical parts, such as the pump, is reduced, e.g. unnecessary activation of mechanical parts are reduced, thereby reducing mechanical wear.

The audiologic test apparatus and/or the audiologic test system may be configured to perform the disclosed method and/or any steps of the disclosed method.

The disclosed apparatus, system and/or method may be used in performing an audiologic test, e.g. a tympanometric test and/or an otoacoustic emission test.

BRIEF DESCRIPTION OF THE DRAWINGS

The above and other features and advantages of the present invention will become readily apparent to those skilled in the art by the following detailed description of exemplary embodiments thereof with reference to the attached drawings, in which:

Fig. 1     schematically illustrates an exemplary audiologic test system,

Fig. 2     schematically illustrates an exemplary audiologic test system,

Fig. 3     schematically illustrates an exemplary audiologic test system,

Fig. 4     schematically illustrates an exemplary audiologic test system,

Fig. 5     schematically illustrates an exemplary audiologic test apparatus,

Fig. 6     schematically illustrates an exemplary test probe,

Fig. 7     is a flow diagram of a method for detecting insertion of a probe into an ear canal,

Fig. 8     is a flow diagram of a method for detecting insertion of a probe into an ear canal, and,

Fig. 9     shows volume equivalents at different frequencies for a sealed cavity and a cavity with a leak

DETAILED DESCRIPTION

The figures are schematic and simplified for clarity, and they merely show details which are essential to the understanding of the invention, while other details have been left out. Throughout, the same reference numerals are used for identical or corresponding parts.

The first signal has a first primary frequency component at a first primary frequency, e.g. centered around or at a first primary frequency. The first signal may be a pure tone signal with the first primary frequency. The first signal may be an audio signal. The first primary frequency is in the range from 30 Hz to 80 Hz. The first primary frequency may be 40 Hz or 20 Hz. The first signal may be transmitted whenever the apparatus and/or system is switched on or a test procedure is started.

The first signal may comprise a first secondary frequency component at a first secondary frequency, e.g. centered around or at a first secondary frequency. The first secondary frequency may be lower than 180 Hz, such as lower than 150 Hz, such as lower than 100 Hz, such as lower than 40 Hz. The first secondary frequency may be in the range from 15 Hz to 150 Hz, such as in the range from 30 Hz to 80 Hz. The first secondary frequency may be 60 Hz or 40 Hz.

The first response signal may at least partly be or comprise a reflection of the first signal. For example, the first response signal is a reflection of the first signal by a tympanic membrane in an ear canal.

The first insertion criterion is based on the first signal and/or the first response signal, e.g. one or more properties, such as time delay, power ratio, amplitude ratio, cross correlation, or admittance of the first signal and/or the first response signal. For example, the first insertion criterion may be satisfied if a predetermined degree of reflection of the first signal is detected in the first response signal.

The first insertion criterion may be based on a measured admittance based on the first signal and/or the first response signal. The measured admittance may be indicative of or equivalent to a volume. The first insertion criterion may be based on a threshold of the measured admittance and/or the volume indicative of or equivalent to the measured admittance.

Conventionally, an equivalent volume in ml is equal to the measured admittance in mmho when measured with a frequency of 226 Hz. The volume equivalent may be defined to be a volume in ml equal to the admittance in mmho measured with a frequency of 226 Hz. The volume equivalent measured with other frequencies may be:

$$V_{EQ} = Y \cdot \frac{\alpha}{f},$$

wherein $V_{EQ}$ is the volume equivalent in ml, $Y$ is the admittance measured in mmho, f is the frequency measured in Hz, and $\alpha$ is a constant. The constant $\alpha$ can be set to 226 for the calculated $V_{EQ}$ to be comparable to a conventional volume equivalent measured with a frequency of 226.

The first insertion criterion may be satisfied if the volume equivalent is less than 10 ml.

The audiologic test may be initiated if the first insertion criterion is satisfied. Initiation of the audiologic test may depend on a plurality of insertion criteria or insertion tests, e.g. the first insertion criterion and a second insertion criterion. The audiologic test may be initiated automatically if the first insertion criterion is satisfied. The audiologic test comprises generating a second signal with a second primary frequency component at a second primary frequency, e.g. centered around or at a second primary frequency. The apparatus may be configured to stop or turn of generation of the first signal if the first insertion criterion is satisfied.

The second signal has a second primary frequency component at a second primary frequency, e.g. centered around or at a second primary frequency. The second signal may be an audio signal. The second primary frequency may be in the range from 190 Hz to 250 Hz, such as 200 Hz or 226 Hz, such as conventionally used for audiologic tests, such as tympanometry. Alternatively, the second primary frequency may be in the range from 950 Hz to 1050 Hz, such as conventionally used for audiologic tests of small children. The first primary frequency may be lower or substantially lower than the second primary frequency. For example, the difference between the first primary frequency and the second primary frequency may be at least 50 Hz, such as at least 100 Hz.

The first signal and/or the second signal may be a pure tone signal or substantially a pure tone signal, e.g. a signal consisting of a single frequency component or substantially a single frequency component.

Before initiating the audiologic test, the method may comprise initiating/performing a second insertion test, e.g. if the first criterion is satisfied or fulfilled or a first insertion test is passed. Before initiating the audiologic test, the method may comprise, e.g. as a part of the second insertion test, modifying pressure (in the ear canal) if the first insertion criterion is satisfied or the first insertion test is passed. The method may further comprise, e.g. as a part of the second insertion test, detecting a pressure response. Furthermore, the method may comprise, e.g. as a part of the second insertion test, determining if a second insertion criterion is satisfied. The second insertion criterion may be based on the pressure response and/or the pressure modification. The audiologic test may be initiated if the first and second insertion criterions are satisfied and/or if the first insertion test and the second insertion test are passed.

The audiologic test apparatus comprises a housing. The housing may enclose one or more components of the audiologic test apparatus, e.g. the processing unit. The audiologic test apparatus may comprise one or more interfaces, such as the probe interface, for communication between components enclosed in the housing and components and/or users external to the audiologic test apparatus. The housing may be a metal housing, a plastic housing and/or a combination of a metal and plastic housing.

The audiologic test apparatus comprises a processing unit. The processing unit may comprise a microprocessor, an analogue-to-digital converter (ADC), and/or a memory module. The processing unit may be configured to perform signal analysis of one or more input signals, such as the first response signal. The processing unit may be configured to provide one or more output signals, such as control signals. For example, the processing unit may provide a control signal to the tone generator, such as a first and/or second control signal, whereby the tone generator is instructed to generate an electrical signal, such as the first electrical signal and/or the second electrical signal.

The audiologic test system may comprise a tone generator. The tone generator of the test system may be a tone generator of the audiologic test apparatus. The tone generator may be configured to generate an electrical signal, such as the first electrical signal, or such as the second electrical signal. The electrical signal may be representative of a signal with a primary frequency component at a primary frequency, such as the first signal with the first primary frequency component at a first primary frequency, and/or such as the second signal with the second primary frequency component at the second primary frequency. The tone generator may be connected to one or more speakers, such as the first speaker and/or a second speaker, wherein the one or more speakers converts the electrical signal(s) of the tone generator to the signal represented by the electrical signal.

The audiologic test apparatus may comprise a probe interface. The probe interface may provide a possibility of connecting a probe to the audiologic test apparatus. The probe interface may comprise one or more electrical connectors and/or one or more fluid communication channels, such as a pump port. The one or more electrical connectors may provide electrical communication between components of the audiologic test apparatus and components of the probe. For example, the electrical connectors may provide electrical communication between the tone generator and a speaker, such as the first speaker, and/or between the processing unit and a microphone, such as the first microphone. The one or more fluid communication channels and/or pump ports may provide fluid communication between the probe and a pump module of the test apparatus, e.g. in order to adjust pressure in the ear canal of a test person.

The test probe may be connectable to the audiologic test apparatus. Alternatively and/or additionally, the test probe may be connected to the audiologic test apparatus.

The test probe may comprise one or more parts including a first part and/or a second part. A part of the test probe, such as the first part, may be configured for insertion into an ear canal. The test probe and/or the first part of the test probe may be adapted to receive a disposable and/or flexible tip. The disposable and/or flexible tip may be designed to perform an air tight seal of the ear canal. A disposable tip may prevent requirement of sterilizing the probe because the disposable tip may be sterile and disposed of after use. The tip may facilitate adaption of the test probe for different ear canal geometries/sizes.

The audiologic test system may comprise one or more speakers, such as the first speaker and/or a second speaker. The audiologic test apparatus may comprise one or more speakers, such as the first speaker and/or the second speaker. The test probe may comprise one or more speakers, such as the first speaker and/or the second speaker.

The first speaker and/or second speaker may be connected and/or connectable to the tone generator, e.g. via the probe interface. The first signal may be generated by the first speaker and/or the second speaker. The second signal may be generated by the first speaker and/or the second speaker. The first signal may be generated by the first speaker and the second signal may be generated by the second speaker.

The audiologic test apparatus may comprise a microphone, such as the first microphone. The test probe may comprise a microphone, such as the first microphone. The first microphone may be connected and/or connectable to the processing unit, e.g. via the probe interface.

The apparatus/system may comprise a display, such as an LCD display, such as an LED display, such as an OLED display. The apparatus, such as the housing of the apparatus, may comprise the display.

The first primary frequency component may be at a lower cut-off frequency of the tone generator. For example, the first primary frequency may be the lowest frequency that the tone generator can generate. It may be beneficial to use the lowest possible frequency for detecting enclosure of the probe/correct positioning of the test probe in the ear canal.

The audiologic test apparatus may comprise a pump module. The pump module may be connected to the processing unit. The pump module may have a port in fluid communication with the pump port of the probe interface. The audiologic test apparatus, e.g. the pump module, and/or the test probe may comprise a pressure sensor. The pressure sensor may be configured to measure the actual pressure in the ear canal.

The audiologic test may comprise modifying the pressure in the ear canal, and the pressure in the ear canal may be modified and/or sensed by the use of the pump module.

The apparatus and/or system may be configured to perform a second insertion test. The second insertion test may be based on generation and detection of pressure/pressure changes in the ear canal. The second insertion test may be a leakage test.

The apparatus and/or system may be configured to modify pressure if the first insertion criterion is satisfied. The apparatus and/or system may furthermore be configured to detect a pressure response. The apparatus and/or system may furthermore be configured to determine if a second insertion criterion is satisfied. The second insertion criterion may be based on the pressure response. The apparatus and/or system may furthermore be configured to initiate the audiologic test if the first and second insertion criterions are satisfied.

The pump module may be configured to modify the pressure if the first insertion criterion is satisfied. The pressure sensor may be configured to detect the pressure response. The processing unit may be configured to determine if the second insertion criterion is satisfied, e.g. the processing unit may be configured to analyze the pressure response to determine if the second insertion criterion is satisfied. The processing unit may furthermore initiate the audiologic test if the first and second insertion criterions are satisfied.

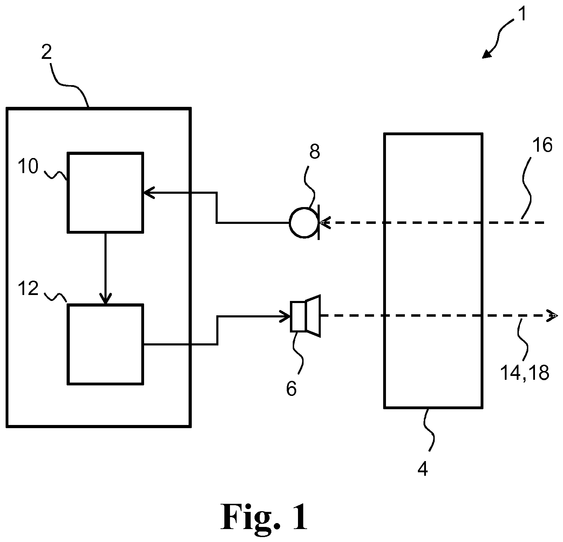

Fig. 1 schematically illustrates an exemplary audiologic test system 1 for performing an audiologic test. The audiologic test system 1 comprises an audiologic test apparatus 2, a test probe 4, a first speaker 6, and a first microphone 8. The audiologic test apparatus comprises a processing unit 10, and a tone generator 12 connected to the processing unit 10. The first microphone 8 is connected to the processing unit 10, and the first speaker is connected to the tone generator 12.

The audiologic test system 1 is configured to generate a first signal 14 with a first primary frequency component at a first primary frequency, and receive a first response signal 16. The audiologic test system 1 generates the first signal 14 by the first speaker 6 and transmits the first signal 14 via the test probe 4. The audiologic test system 1 receives the first response signal 16 by the first microphone 8 via the test probe 4.

The audiologic test system 1 is further configured to determine if a first insertion criterion is satisfied, wherein the first insertion criterion is based on the first signal 14 and the first response signal 16. The audiologic test system 1 is further configured to initiate the audiologic test if at least the first insertion criterion is satisfied. The audiologic test comprises that the audiologic test system 1 generates a second signal 18 with a second primary frequency component at a second primary frequency and measuring a reflection from e.g. a tympanic membrane in an ear canal. The audiologic test system 1 generates the second signal 18 by the first speaker 6 and transmits the second signal 18 via the test probe 4.

The first signal 14 and the second signal 18 differ at least by their primary frequencies at different primary frequencies. Hence, the first primary frequency is lower than the second primary frequency. The first primary frequency is within a range from 30 Hz to 80 Hz. The second primary frequency may be in the range above approximately 200 Hz such as in the range between 190 Hz and 250 Hz, e.g. with second primary frequency between 200 Hz and 230 Hz, such as 200 Hz or 226 Hz or in the range of approximately 900 Hz - 1100 Hz such as 1000Hz. In an exemplary apparatus, the second primary frequency of second signal 18 may be chosen in the range of 180 - 250 Hz for persons to be tested aged approximately 6 months and above, and the second primary frequency of second signal 18 may be chosen in the range 950 Hz - 1050 Hz for persons to be tested aged below approximately 6 months.

The first response signal 16 may at least partly be a reflection of the first signal 14. Analysis of the first response signal 16 may indicate that a probe part is inserted into a cavity, e.g. an ear canal. The first insertion criterion may, for example, be satisfied if a predetermined degree of reflection of the first signal 14 is found in the first response signal 16.

The first primary frequency component at low frequency provides an improved leakage detection than using a higher frequency, e.g. a frequency equivalent to the second primary frequency.

The determination of the first insertion criterion may be computed by the processing unit 10. The processing unit 10 may control the tone generator to generate the first and/or second signal via the first speaker 6. The processing unit may be configured to initiate the audiologic test if the at least first insertion criterion is satisfied.

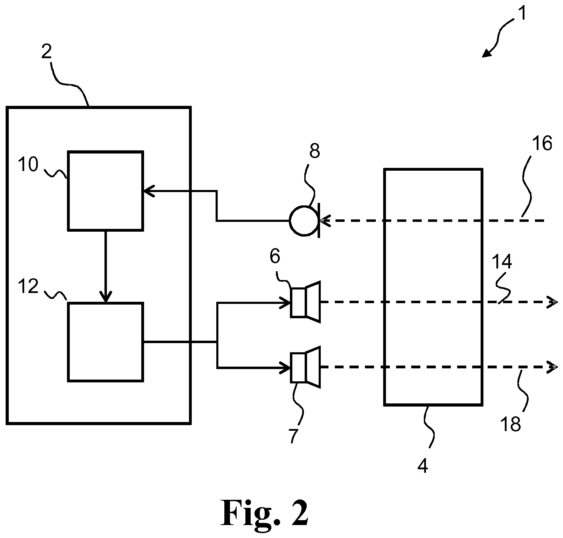

Fig. 2 schematically illustrates an exemplary audiologic test system 1' for performing an audiologic test. The audiologic test system 1' is similar to the audiologic test system 1 of Fig. 1. However, the audiologic test system 1' comprises a second speaker 7, and the audiologic test system 1' is configured to generate the second signal 18 by the second speaker 7.

The first speaker 6 may be configured to generate the first signal 14 with the first primary frequency component at the first primary frequency, and the second speaker 7 may be configured to generate the second signal 18 with the second primary frequency component at the second primary frequency. In an exemplary system (not shown) the tone generator 12 may be omitted by utilizing designated first and second speakers that are specifically configured to transmit a signal having the first and second primary frequency component, respectively.

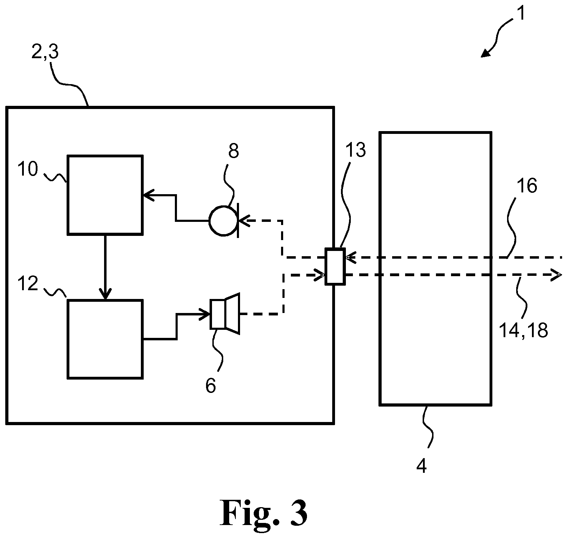

Fig. 3 schematically illustrates an exemplary audiologic test system 1 for performing an audiologic test, wherein the audiologic test apparatus 2 comprises a housing 3, the first speaker 6 and the first microphone 8. The first speaker 6 and the first microphone 8 is comprised within the housing 3 of the audiologic test apparatus. Furthermore the audiologic test apparatus 2 comprises a probe interface 13 for connecting the test probe 4 to the audiologic test apparatus 2.

The first speaker 6 generates the first and/or second signal 14, 18. The first signal 14 and/or second signal 18 is transmitted through the probe interface 13 and the probe 4. Similarly, the first response signal 16 is received via the probe 4 and transmitted through the probe interface 13 to the microphone in the housing 3 of the audiologic test apparatus 2.

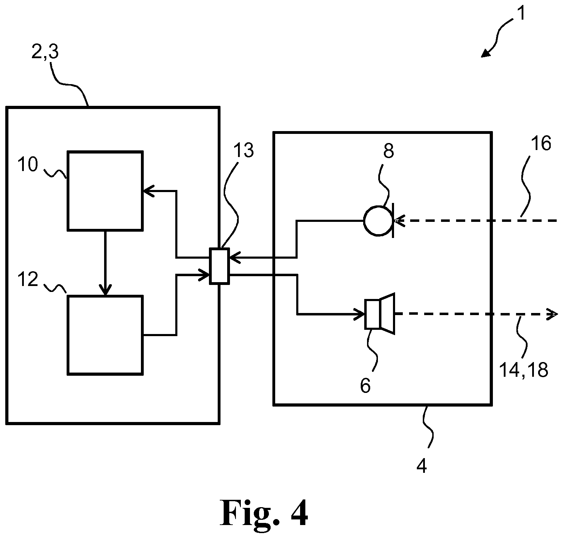

Fig. 4 schematically illustrates an exemplary audiologic test system 1 for performing an audiologic test, wherein the test probe 4 comprises, the first speaker 6 and the first microphone 8. Furthermore the audiologic test apparatus 2 comprises a probe interface 13 for connecting the test probe 4 to the audiologic test apparatus 2.

A first and/or second electrical signal representative of the first and/or second signal 14, 18 is transmitted from the audiologic test apparatus 2 through the probe interface 13 to the first speaker 6 in the test probe 4. In response to receiving the first and/or second electrical signal, the first speaker 6 generates the first signal 14 and/or second signal 18 in the test probe 4. Similarly, the first response signal 16 is received by the first microphone 8 in the test probe. The first microphone 8 transmits a first electrical response signal representative of the first response signal 16 through the probe interface 13 to the audiologic test apparatus 2.

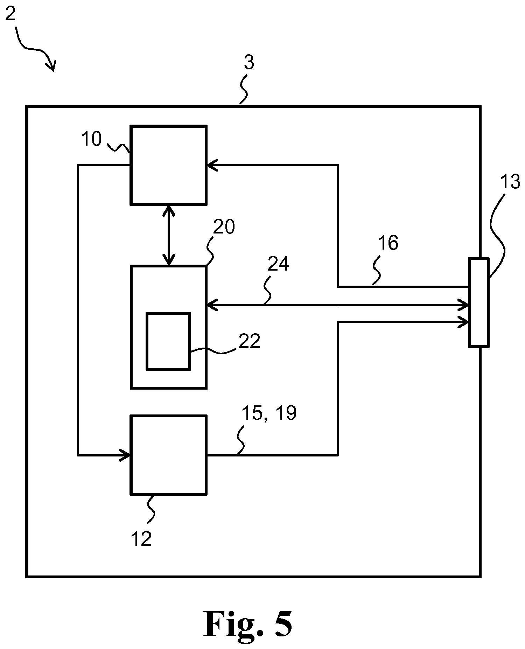

Fig. 5 schematically illustrates an exemplary audiologic test apparatus 2 for performing an audiologic test. The audiologic test apparatus 2 comprises a housing 3, a processing unit 10, and a tone generator 12.

The tone generator 12 generates a first electrical signal 15 representative of the first signal 14 (Figs. 1-4). The first and/or second speaker 6, 7 (Figs. 1-4) receives the first electrical signal 15 and generates the first signal 14. The tone generator furthermore generates a second electrical signal 19 representative of the second signal 18 (Figs. 1-4). The first speaker 6 and/or the second speaker 7 receives the second electrical signal 19 and generates the second signal 18.

Generation of the first and second electrical signal 15, 19 may be simultaneous or differentiated in time. The second electrical signal 19 is at least generated during the audiologic test.

The exemplary audiologic test apparatus 2 as depicted in Fig. 5 furthermore comprises a pump module 20. The pump module 20 is in fluid connection 24 with a pump port of the probe interface 13. Hence, when a test probe is connected to the pump interface, and the probe is inserted into an ear canal (not shown), the pump module 20 may be in fluid connection with the ear canal.

The pump module 20 furthermore comprises a pressure sensor 22. The pressure sensor 22 is, in the depicted example, comprised within the pump module 20.

However, the pressure sensor 22 may be positioned anywhere where it is able to detect the pressure in the ear canal. For example, in an alternative exemplary apparatus, the pressure sensor 22 is positioned in the probe interface 13.

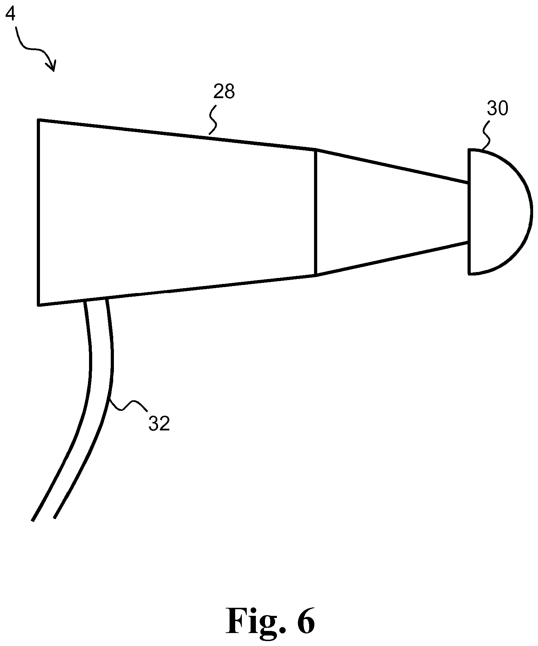

A test probe 4 (Fig. 6) is connectable to the audiologic test apparatus via the probe interface 13. The probe interface 13 may comprise one or more electrical connectors and/or one or more fluid communication channels.

Fig. 6, schematically illustrates an exemplary test probe 4. The test probe 4 comprises a probe housing 28, and a first probe part 30. Fig. 6 furthermore illustrates a probe connector 32 for connecting the test probe 4 with an audiologic test apparatus. The first probe part 30 is adapted for insertion into an ear canal of a person (not shown).

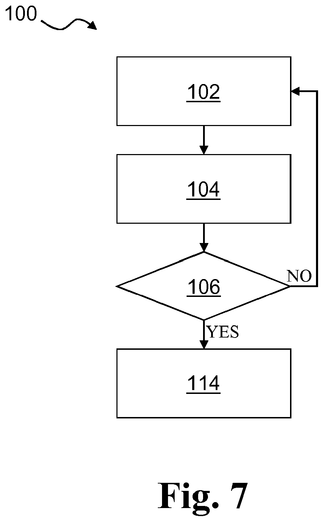

Fig. 7 is a flow diagram of a method 100 for detecting insertion of a probe into an enclosure, such as an ear canal. The method 100 comprises: generating 102 a first signal with a first primary frequency component at a first primary frequency; detecting 104 a first response signal; determining 106 if a first insertion criterion is satisfied; and initiating 114 an audiologic test.

The first insertion criterion may be based on the first signal generated 102 and the first response signal detected 104. The first insertion criterion may be indicative of whether the probe is inserted into a cavity, such as an ear canal. The first insertion criterion may be indicative of whether the probe provides a seal between the probe and the ear canal, such as between the probe and the wall of the ear canal. If determination 106 of the first insertion criterion yields that the first insertion criterion is satisfied, the method continues to initiation 114 of the audiologic test. If determination 106 of the first insertion criterion yields that the first insertion criterion is not satisfied, the method returns to the beginning of the method 100, i.e. generation 102 of the first signal. Furthermore, if the first insertion criterion is not satisfied, the method 100 may comprise signaling to reposition the probe, before returning to the beginning of the method 100. Signaling may be audible and/or visible signals to the operator.

The first signal has a first primary frequency component at a first primary frequency. The first primary frequency is within a range from 30 Hz to 80 Hz.

The audiologic test comprises transmitting a second signal. The second signal has a second primary frequency component at a second primary frequency. The second primary frequency may be in the range of 190-250 Hz, such as in the range of 200-230 Hz, such as 220 or 226 Hz.

The first primary frequency of the first acoustic signal is lower than the second primary frequency of the second signal. The low first primary frequency provides a better detection of enclosure or sealing than using a higher frequency, e.g. a frequency equivalent to the second primary frequency. Thus, by detecting enclosure and/or leakage using a low frequency signal, such as the first signal having a first primary frequency component at a first primary frequency, the risk of falsely detecting an enclosure is decreased. Thereby, activation of the pump while unable to increase the pressure due to leaks or while the system is not ready for testing may be avoided.

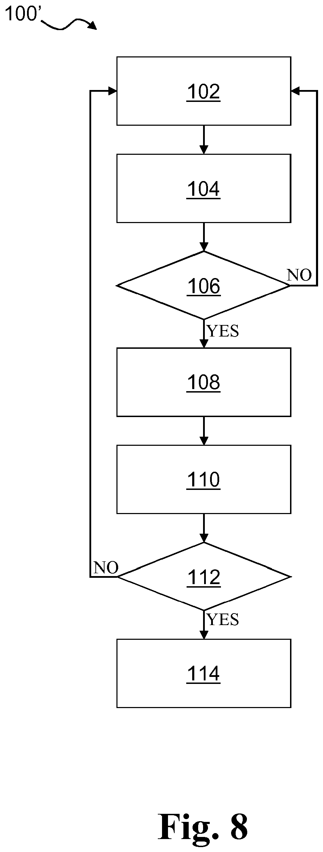

Fig. 8 is a flow diagram of a method 100' for detecting insertion of a probe into an enclosure, such as an ear canal. The method 100' comprises the same initial steps 102, 104, 106 as the method 100 shown in Fig. 7. The method 100' furthermore comprises modifying 108 the pressure in the ear canal, detecting 110 a pressure response, and determining 112 if a second insertion criterion is satisfied.

Modification 108 of the pressure may comprise increasing and/or decreasing the pressure. Detection 110 of the pressure response may provide information of the current pressure, e.g. relative to atmospheric pressure. Detection 110 of the pressure response provides information of whether the modification 108 of the pressure perform as intended, i.e. does the pressure rise and/or fall as expected? The pressure measurement may additionally be associated with the reflected signal, e.g. the first response signal 16 (Figs. 1-5) to e.g. provide a reflex-measurement.

The second insertion criterion is based on the pressure response. The second insertion criterion is indicative of whether the probe is properly inserted into a cavity, such as an ear canal, and if an appropriate air tight seal is created. The second insertion criterion may be that the detected 110 pressure corresponds sufficiently to the modification 108 of the pressure. If determination 112 of the second insertion criterion yields that the second insertion criterion is satisfied, the method continues to initiation 114 of the audiologic test. If determination 112 of the second insertion criterion yields that the second insertion criterion is not satisfied, the method returns to the beginning of the method, i.e. generation 102 of the first signal. Alternatively, if determination 112 of the second insertion criterion yields that the second insertion criterion is not satisfied, the method may return to modification 108 of the pressure, e.g. because steps 102-106 have indicated a seal. Furthermore, if the second insertion criterion is not satisfied, the method 100 may comprise signaling to reposition the probe, before returning to the beginning of the method 100 and/or to modification 108 of the pressure. Signaling may be audible and/or visible signals to the operator.

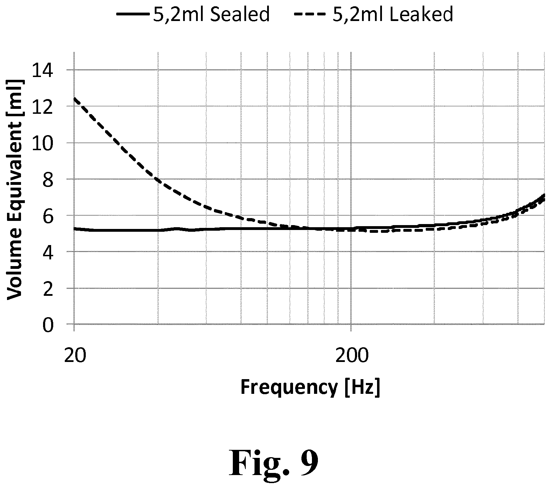

Fig. 9 shows test results for a test of detecting enclosure utilizing signals having different frequencies. The horizontal axis shows the frequency in Hz, such as the first primary frequency component of the first signal. The vertical axis shows the volume equivalent measured. The volume equivalent is derived from measuring admittance and applying the formula:

$$\text{Volume equivalent [ml]} = \text{admittance [mmho]} * 226 / \text{frequency [Hz]}.$$

Two tests were performed with a cavity having a volume of 5.2 ml. The cavity had a small leak which could be opened or closed to imitate an air tight cavity and a leaking cavity.

For different frequencies, the solid line shows volume equivalents measured in a 5.2 ml sealed cavity, and the dashed line shows volume equivalents measured in a 5.2 ml cavity with a leak. The solid line shows that the volume equivalent is measured to be around 5.2 ml for all low frequencies, e.g. below 500 Hz, whereas the dashed line shows that for decreasing frequencies, the volume equivalent is increasing.

Comparing the results for the conventional used 226 Hz, the volume equivalent is measured to be the same in the sealed (solid line) and leaked (dashed line) cavity. This result implies that in this setup it cannot be determined from measuring using a first signal with a first primary frequency component of 226 Hz whether or not the cavity is sealed or leaked. However lowering the first primary frequency component of the first signal, e.g. to 20 Hz, the volume equivalent of the leaked cavity (dashed line) is measured to be approximately 12.5 ml. Hence, the risk of falsely detecting a sealed cavity where the cavity actually has a leakage is heavily reduced by the method and apparatus disclosed herein.

LIST OF REFERENCES

| 1 | audiologic test system |
|---|---|
| 2 | audiologic test apparatus |
| 3 | housing |
| 4 | test probe |
| 6 | first speaker |
| 7 | second speaker |
| 8 | first microphone |
| 10 | processing unit |
| 12 | tone generator |
| 13 | probe interface |
| 14 | first signal |
| 15 | first electrical signal |
| 16 | first response signal |
| 18 | second signal |
| 19 | second electrical signal |
| 20 | pump module |
| 22 | pressure sensor |
| 24 | fluid communication |
| 28 | probe housing |
| 30 | probe first part |
| 32 | probe connector |
| 100, 100' | method |
| 102 | generating first signal |
| 104 | detecting response signal |
| 106 | determining first insertion criterion |
| 108 | modifying pressure |
| 110 | detecting pressure response |
| 112 | determining second insertion criterion |

114        initiating audiologic test

## Claims

**1.**  An audiologic test apparatus (2) for performing an audiologic test in an ear canal, the audiologic test apparatus (2) comprising:

a housing (3),
a processing unit (10),
a tone generator (12) connected to the processing unit (10), and
a probe interface (13) for connecting the audiologic test apparatus (2) to a test probe;
wherein the apparatus is configured to:

generate by the tone generator (12) a first electrical signal (15) representative of a first signal (14) with a first primary frequency component at a first primary frequency;
receive a first response signal (16);
determine if a first insertion criterion is satisfied, wherein the first insertion criterion is based on the first signal (14) and the first response signal (16); and
initiate the audiologic test if at least the first insertion criterion is satisfied, the audiologic test comprising generating a second electrical signal (19) representative of a second signal (18) with a second primary frequency component at a second primary frequency;
**characterised by** the first primary frequency being within a range from 30 Hz to 80 Hz and lower than the second primary frequency.

**2.**  Audiologic test apparatus according to claim 1, wherein the first primary frequency component comprises a lower cut-off frequency of the tone generator (13) i.e. wherein the first primary frequency is the lowest frequency that the tone generator (12) may generate.

**3.**  Audiologic test apparatus according to any of claims 1 or 2, the apparatus comprising a pump module (20) connected to the processing unit (10) and having a port in fluid communication with a pump port of the probe interface (13), wherein the audiologic test comprises modifying the pressure in the ear canal.

**4.**  Audiologic test apparatus according to claim 3, wherein the pump module (20) comprises a pressure sensor.

**5.**  Audiologic test apparatus according to any of the preceding claims, wherein the apparatus is configured to:

modify pressure if the first insertion criterion is satisfied;
detect a pressure response;
determine if a second insertion criterion is satisfied, wherein the second insertion criterion is based on the pressure response; and
initiate the audiologic test if the first and second insertion criterion are satisfied.

**6.**  Audiologic test apparatus according to any of the preceding claims, wherein the second primary frequency is in the range from 190 Hz to 250 Hz or in the range from 950 Hz to 1050 Hz.

**7.**  A method (900) for performing an audiologic test, the method (100) comprising:

generating (102) a first signal (114) with a first primary frequency component at a first primary frequency;
detecting (104) a first response signal (16);
determining if a first insertion criterion is satisfied, wherein the first insertion criterion is based on the first signal (14) and the first response signal (16); and
initiating (114) the audiologic test if at least the insertion criterion is satisfied, the audiologic test comprising generating a second signal (18) with a second primary frequency component at a second primary frequency;
the first primary frequency being within a range from 30 Hz to 80 Hz and lower than the second primary frequency.

**8.**  Method according to claim 7, wherein the method before initiating the audiologic test comprises:

modifying (108) pressure if the first insertion criterion is satisfied;
detecting (110) a pressure response;
determining (112) if a second insertion criterion is satisfied, wherein the second insertion criterion is based on the pressure response; and
initiating (114) the audiologic test if the first and second insertion criterion are satisfied.

9. Method according to any of claims 7-8, wherein the second primary frequency is in the range from 190 Hz to 250 Hz or in the range from 950 Hz to 1050 Hz.

**Patentansprüche**

1. Audiologische Testvorrichtung (2) zur Durchführung eines audiologischen Tests in einem Gehörgang, wobei die audiologische Testvorrichtung (2) Folgendes umfasst:

   ein Gehäuse (3),
   eine Verarbeitungseinheit (10),
   einen mit der Verarbeitungseinheit (10) verbundenen Tongenerator (12), und
   eine Sondenschnittstelle (13) zum Verbinden der audiologischen Testvorrichtung (2) mit einer Testsonde;
   wobei die Vorrichtung so konfiguriert ist, dass sie:

      durch den Tongenerator (12) ein erstes elektrisches Signal (15) erzeugt, das ein erstes Signal (14) mit einer ersten primären Frequenzkomponente bei einer ersten primären Frequenz darstellt;
      ein erstes Antwortsignal (16) empfängt;
      feststellt, ob ein erstes Einführungskriterium erfüllt ist, wobei das erste Einführungskriterium auf dem ersten Signal (14) und dem ersten Antwortsignal (16) basiert; und
      den audiologischen Test einleitet, wenn zumindest das erste Einführungskriterium erfüllt ist, wobei der audiologische Test Erzeugen eines zweiten elektrischen Signals (19) umfasst, das ein zweites Signal (18) mit einer zweiten primären Frequenzkomponente bei einer zweiten primären Frequenz darstellt;

   **dadurch gekennzeichnet, dass** die erste primäre Frequenz innerhalb eines Bereichs von 30 Hz bis 80 Hz liegt und niedriger als die zweite primäre Frequenz ist.

2. Audiologische Testvorrichtung nach Anspruch 1, wobei die erste primäre Frequenzkomponente eine untere Grenzfrequenz des Tongenerators (13) umfasst, d. h. wobei die erste primäre Frequenz die niedrigste Frequenz ist, die der Tongenerator (12) erzeugen kann.

3. Audiologische Testvorrichtung nach einem der Ansprüche 1 oder 2, wobei die Vorrichtung ein Pumpmodul (20) umfasst, das mit der Verarbeitungseinheit (10) verbunden ist und einen Anschluss aufweist, der in Fluidverbindung mit einem Pumpenanschluss der Sondenschnittstelle (13) steht, wobei der audiologische Test Modifizieren des Drucks im Gehörgang umfasst.

4. Audiologische Testvorrichtung nach Anspruch 3, wobei das Pumpmodul (20) einen Drucksensor umfasst.

5. Audiologische Testvorrichtung nach einem der vorhergehenden Ansprüche, wobei die Vorrichtung so konfiguriert ist, dass sie: Druck modifiziert, wenn das erste Einführungskriterium erfüllt ist;

   eine Druckreaktion erkennt;
   feststellt, ob ein zweites Einführungskriterium erfüllt ist, wobei das zweite Einführungskriterium auf der Druckreaktion basiert; und
   den audiologischen Test einleitet, wenn das erste und das zweite Einführungskriterium erfüllt sind.

6. Audiologische Testvorrichtung nach einem der vorhergehenden Ansprüche, wobei die zweite primäre Frequenz in dem Bereich von 190 Hz bis 250 Hz oder in dem Bereich von 950 Hz bis 1050 Hz liegt.

7. Verfahren (900) zur Durchführung eines audiologischen Tests, wobei das Verfahren (100) Folgendes umfasst:

   Erzeugen (102) eines ersten Signals (114) mit einer ersten primären Frequenzkomponente bei einer ersten primären Frequenz;

Erkennen (104) eines ersten Antwortsignals (16);

Feststellen, ob ein erstes Einführungskriterium erfüllt ist, wobei das erste Einführungskriterium auf dem ersten Signal (14) und dem ersten Antwortsignal (16) basiert; und

Einleiten (114) des audiologischen Tests, wenn zumindest das Einfügungskriterium erfüllt ist, wobei der audiologische Test Erzeugen eines zweiten Signals (18) mit einer zweiten primären Frequenzkomponente bei einer zweiten primären Frequenz umfasst;

wobei die erste primäre Frequenz innerhalb eines Bereichs von 30 Hz bis 80 Hz liegt und niedriger ist als die zweite primäre Frequenz.

8. Verfahren nach Anspruch 7, wobei das Verfahren vor dem Einleiten des audiologischen Tests Folgendes umfasst:

Modifizieren (108) von Druck, wenn das erste Einführungskriterium erfüllt ist;

Erkennen (110) einer Druckreaktion;

Feststellen (112), ob ein zweites Einführungskriterium erfüllt ist, wobei das zweite Einführungskriterium auf der Druckreaktion basiert; und

Einleiten (114) des audiologischen Tests, wenn das erste und das zweite Einführungskriterium erfüllt sind.

9. Verfahren nach einem der Ansprüche 7-8, wobei die zweite primäre Frequenz im Bereich von 190 Hz bis 250 Hz oder im Bereich von 950 Hz bis 1050 Hz liegt.

## Revendications

1. Appareil de test audiophonatoire (2) permettant d'effectuer un test audiophonatoire dans un conduit auditif, l'appareil de test audiophonatoire (2) comprenant :

un boîtier (3),

une unité de traitement (10),

un générateur de tonalité (12) relié à l'unité de traitement (10), et

une interface de sonde (13) permettant de relier l'appareil de test audiophonatoire (2) à une sonde de test ;

dans lequel l'appareil est configuré pour :

générer au moyen du générateur de tonalité (12) un premier signal électrique (15) représentatif d'un premier signal (14) avec une première composante de fréquence principale à une première fréquence principale ;

recevoir un premier signal de réponse (16) ;

déterminer si un premier critère d'insertion est satisfait, dans lequel le premier critère d'insertion est basé sur sur le premier signal (14) et le premier signal de réponse (16) ; et

lancer le test audiophonatoire si au moins le premier critère d'insertion est satisfait, le test audiophonatoire comprenant la génération d'un second signal électrique (19) représentatif d'un second signal (18) avec une seconde composante de fréquence principale à une seconde fréquence principale ;

**caractérisé en ce que** la première fréquence principale qui se situe dans une plage comprise entre 30 Hz et 80 Hz est inférieure à la seconde fréquence principale.

2. Appareil de test audiophonatoire selon la revendication 1, dans lequel la première composante de fréquence principale comprend une fréquence de coupure inférieure du générateur de tonalité (13), c'est-à-dire dans lequel la première fréquence principale est la fréquence la plus basse que le générateur de tonalité (12) peut générer.

3. Appareil de test audiophonatoire selon l'une quelconque des revendications 1 ou 2, l'appareil comprenant un module de pompe (20) relié à l'unité de traitement (10) et ayant un orifice en communication fluidique avec un orifice de pompe de l'interface de sonde (13), dans lequel le test audiophonatoire comprend la modification de la pression dans le conduit auditif.

4. Appareil de test audiophonatoire selon la revendication 3, dans lequel le module de pompe (20) comprend un capteur de pression.

5. Appareil de test audiophonatoire selon l'une quelconque des revendications précédentes, dans lequel l'appareil est configuré pour :

modifier la pression si le premier critère d'insertion est satisfait ;

détecter une réponse de pression ;

déterminer si un second critère d'insertion est satisfait, dans lequel le second critère d'insertion est basé sur la réponse de pression ; et

lancer le test audiophonatoire si le premier et le second critères d'insertion sont satisfaits.

6. Appareil de test audiophonatoire selon l'une quelconque des revendications précédentes, dans lequel la seconde fréquence principale se situe dans la plage de 190 Hz à 250 Hz ou dans la plage de 950 Hz à 1 050 Hz.

7. Procédé (900) permettant d'effectuer un test audiophonatoire, le procédé (100) comprenant :

la génération (102) d'un premier signal (114) avec une première composante de fréquence principale à une première fréquence principale ;

la détection (104) d'un premier signal de réponse (16) ;

le fait de déterminer si un premier critère d'insertion est satisfait, dans lequel le premier critère d'insertion est basé sur le premier signal (14) et le premier signal de réponse (16) ; et

le lancement (114) du test audiophonatoire si au moins le critère d'insertion est satisfait, le test audiophonatoire comprenant la génération d'un second signal (18) avec une seconde composante de fréquence principale à une seconde fréquence principale ;

dans lequel la première fréquence principale qui se situe dans une plage comprise entre 30 Hz et 80 Hz est inférieure à la seconde fréquence principale.

8. Procédé selon la revendication 7, dans lequel le procédé avant de lancer le test audiophonatoire comprend :

la modification (108) de la pression si le premier critère d'insertion est satisfait ;

la détection (110) d'une réponse de pression ;

le fait de déterminer (112) si un second critère d'insertion est satisfait, dans lequel le second critère d'insertion est basé sur la réponse de pression ; et

le lancement (114) du test audiophonatoire si les premier et second critères d'insertion sont satisfaits.

9. Procédé selon l'une quelconque des revendications 7 et 8, dans lequel la seconde fréquence principale se situe dans la plage de 190 Hz à 250 Hz ou dans la plage de 950 Hz à 1 050 Hz.

**Fig. 1**

**Fig. 2**

Fig. 3

**Fig. 4**

Fig. 5

**Fig. 6**

100

```
         ┌─────────────────┐
         │      102        │◄─────┐
         └────────┬────────┘      │
                  │               │
                  ▼               │
         ┌─────────────────┐      │
         │      104        │      │
         └────────┬────────┘      │
                  │               │
                  ▼          NO   │
              ╱───────╲───────────┘
             ╱   106   ╲
             ╲         ╱
              ╲───────╱
                  │ YES
                  ▼
         ┌─────────────────┐
         │      114        │
         └─────────────────┘
```

# Fig. 7

100'

**Fig. 8**

Fig. 9

**EP 2 937 040 B1**

**REFERENCES CITED IN THE DESCRIPTION**

**Patent documents cited in the description**

- US 5577511 A
- US 7050592 B